(19) Europäisches Patentamt
European Patent Office
Office européen des brevets

(11) Veröffentlichungsnummer: **0 062 264**
**A2**

(12) **EUROPÄISCHE PATENTANMELDUNG**

(21) Anmeldenummer: **82102584.8**

(51) Int. Cl.[3]: **C 07 D 213/73**

(22) Anmeldetag: **26.03.82**

(30) Priorität: **31.03.81 CH 2166/81**

(43) Veröffentlichungstag der Anmeldung: **13.10.82 Patentblatt 82/41**

(84) Benannte Vertragsstaaten: **AT BE CH DE FR GB IT LI LU NL SE**

(71) Anmelder: **LONZA AG, Gampel/Wallis (CH)**

(72) Erfinder: **Quarroz, Daniel, Dr., Kleegärtenstrasse 1, Visp (Kanton Wallis) (CH)**

(74) Vertreter: **Von Füner, Alexander, Dr. et al, K.L. Schiff Dr. A. von Funer Dipl.-Ing. P. Strehl Dr. U. Schübel-Hopf Dipl.-Ing. D. Ebbinghaus Dr.-Ing. D. Finck Patentanwälte Mariahilfplatz 2 & 3, D-8000 München 90 (DE)**

(54) **Verfahren zur Herstellung von Amino-Pyridinen.**

(57) Amino-Pyridine werden beim Behandeln von Glutarsäuredinitrilen mit $H_2$ in Gegenwart von Pd-Katalysatoren auf $\alpha$-$Al_2O_3$-Trägern in der Gasphase bei Temperaturen von 260 bis 310 °C hergestellt.

EP 0 062 264 A2

## Verfahren zur Herstellung von Amino-Pyridinen

Die vorliegende Erfindung betrifft die Herstellung von Amino-Pyridinen der allgemeinen Formel

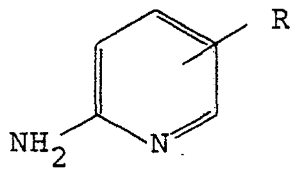

in welcher R ein Wasserstoffatom oder einen niedrigen Alkylrest mit 1 bis 2 C-Atomen bedeutet.

Amino-Pyridine stellen Ausgangsmaterialien für die Herstellung chemotherapeutisch wirksamer Substanzen und pharmakologisch bedeutsamer Verbindungen dar.

Das Verfahren der Erfindung ist dadurch gekennzeichnet, dass man Glutarsäuredinitril der allgemeinen Formel

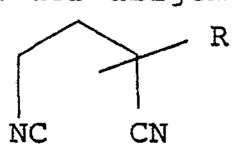

in der R ein Wasserstoffatom oder einen niedrigen Alkylrest mit 1 bis 2 C-Atomen bedeutet, in der Gasphase, zusammen mit Wasserstoff bei Temperaturen von 260 bis 310°C über einen Pt- oder Pd-Katalysator, der auf einen $\alpha$-$Al_2O_3$-Träger aufgebracht ist, leitet.

Neben Glutarsäuredinitril können auch substituierte Glutarsäuredinitrile, vorzugsweise 2-Methylglutarsäuredinitril, zur Anwendung kommen. Eine ganz besonders vorteilhafte Ausgestaltung der Erfindung ist es, wenn 2-Methylglutarsäuredinitril

umgesetzt werden soll, von einem Gemisch von Methylglutarsäuredinitril und Alkylbernsteinsäuredinitril auszugehen, wie es als Nebenprodukt bei der Adipinsäuredinitrilherstellung anfällt.

Das Glutarsäuredinitril wird zweckmässig in einer Menge von 0,1 bis 1,0 g, vorzugsweise von 0,3 bis 0,5 g pro Gramm Katalysator und pro Stunde, eingesetzt.

Die Menge an Wasserstoff liegt vorteilhaft bei 0,5 bis 1,0 Liter Wasserstoff pro Gramm Katalysator und pro Stunde.

Die Reaktionstemperatur liegt vorteilhaft bei 280 bis 300°C.

Als Katalysator kommt Platin oder Palladium zum Einsatz, das in Mengen von 1 bis 10 Gew.-%, vorzugsweise 1 bis 5 Gew.-%, auf einen Träger aus $\alpha$-$Al_2O_3$ aufgebracht wird.

Als $\alpha$-$Al_2O_3$ als Träger kommt zweckmässig solches $\alpha$-$Al_2O_3$ in Frage, das eine spezifische Oberfläche von 100 $m^2/g$ bis 350 $m^2/g$, vorzugsweise 250 $m^2/g$ bis 300 $m^2/g$, aufweist.

Das auf Reaktionstemperatur erhitzte Gemisch aus Glutarsäuredinitril und Wasserstoff wird über oder durch den Katalysator-Träger geleitet. Dabei kann der Katalysator-Träger als Festbett oder als Fliessbett vorliegen.

Das nach dem Verfahren der Erfindung anfallende Reaktionsgemisch besteht aus nichtumgesetztem Ausgangsmaterial, das wieder

zurückgewonnen wird: Aminopyridine und andere Pyridinbasen. Bei Glutarsäuredinitril fallen neben dem 2-Aminopyridin noch grössere Mengen Pyridin und Butyronitril an, die abgetrennt und weiterverwertet werden können. Geht man von Methylglutardinitril aus, fallen neben 2-Amino-3-picolin und 6-Amino-3-picolin noch grössere Mengen 3-Picolin an, die abgetrennt und einem weiteren Verwendungszweck zugeführt werden.

Beispiel 1

210,4 g einer Mischung aus 86,4% 2-Methylglutarsäuredinitril und 13,2% Aetylsuccinodinitril, wie sie bei der Adipinsäuredinitrilsynthese als Nebenprodukt anfällt, wurden bei 280°C zusammen mit 30 Liter $H_2$ pro Stunde während 10 Stunden durch ein Katalysatorbett aus Pd auf $\alpha$-$Al_2O_3$ (5% Pd) in Kugelform gepumpt. Nach Auffangen des Reaktionsproduktes (194,9 g) wurden folgende Produkte isoliert:

23% nicht umgesetztes Methylglutarsäuredinitril
23% 2-Amino-3-picolin
14% 6-Amino-3-picolin
14% Picolin
ferner noch etwa 17% Valero- und 2-Methylbutyronitril.

Für einen Umsatz von ca. 75% erhielt man Aminopicoline in einer Ausbeute von 54% und 3-Picolin in einer Ausbeute von 22%.

Beispiel 2

Aehnlich wie in Beispiel 1, aber bei 310°C, wurden während 6 Stunden Glutarsäuredinitril zusammen mit 20 Liter $H_2$/g Katalysator/h durch den Katalysator gepumpt. Es resultierte 2-Aminopyridin in einer Ausbeute von 26,9%. Daneben wurden 29,9% Pyridin gefunden.

Beispiele 3 - 5

| Beispiel | Edukt | | | $H_2$ | Temp. | Ausbeute % | |
|---|---|---|---|---|---|---|---|
| | Menge g pro g Kat. pro h | ~~Art~~ | | l/h | °C | | |
| | | MGN % | ESN % | | | 3-Picolin | Amino-3-picolin |
| 3 | 0,3 | 87,8 | 11,7 | 10 | 300 | 28,4 | 29,9 |
| 4 | 0,5 | 93 | 7 | 10 | 280 | 48,8 | 47,8 |
| 5 | 0,48 | 86,4 | 13,2 | 30 | 260 | 36,3 | 30,4 |

Beispiel 6

Im Gegensatz zu den Beispielen 1,2,3,4 und 5 kam hier ein Platinkatalysator zum Einsatz: 1% Pt auf einem Träger aus $\alpha$-$Al_2O_3$. Im Prinzip aber wurde gleich wie in vorangehenden Beispielen gearbeitet.

| Edukt | | | $H_2$ | Temp. | Ausbeute % | |
|---|---|---|---|---|---|---|
| Menge g pro g Kat pro h | Art | | l/h | °C | 3-Picolin | Amino-3-picolin |
| | MGN % | ESN % | | | | |
| 0,4 | 100 | -- | 20 | 310 | 11 | 23 |

Patentansprüche

1. Verfahren zur Herstellung von Amino-Pyridinen der allgemeinen Formel

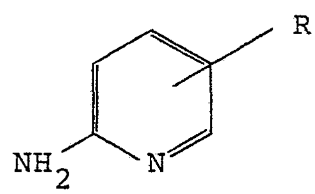

in welcher R einen Wasserstoff oder einen niederen Alkylrest mit 1 bis 2 C-Atomen bedeutet, dadurch gekennzeichnet, dass man das entsprechende Glutarsäuredinitril der allgemeinen Formel

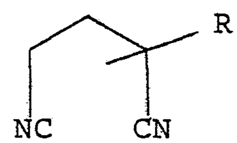

in welcher R die obengenannte Bedeutung aufweist, in der Gasphase, zusammen mit Wasserstoff, bei Temperaturen von 260 bis 310°C, über einen Pt- oder Pd-Katalysator, der auf einem $\alpha$-$Al_2O_3$-Träger aufgebracht ist, leitet.

2. Verfahren nach Patentanspruch 1, dadurch gekennzeichnet, dass man Glutarsäuredinitril in einer Menge von 0,1 bis 1,0 g/g Katalyt/h über den Katalysator führt.

3. Verfahren nach Patentansprüchen 1 und 2, dadurch gekennzeichnet, dass man Wasserstoff in einer Menge von 0,5 bis 1,0 l/g Katalyt/h verwendet.

4. Verfahren nach Patentansprüchen 1 bis 3, dadurch gekennzeichnet, dass der Pt- oder Pd-Katalysator auf einen $\alpha$-$Al_2O_3$-Träger aufgebracht ist, der eine spezifische Oberfläche von 100 $m^2/g$ bis 300 $m^2/g$ aufweist.

5. Verfahren nach Patentansprüchen 1 bis 4, dadurch gekennzeichnet, dass die Porosität des als Träger für den Pt- oder Pd-Katalysator bestimmten $\alpha$-$Al_2O_3$ einen Anteil an Mikroporen von 0,15 $cm^3/g$ und einen Anteil an Makroporen von 0,010 $cm^3/g$ aufweist.